# EUROPEAN PATENT SPECIFICATION

(11) **EP 0 273 223 B1**
(45) Date of publication and mention of the grant of the patent: **10.03.1993**
(21) Application number: 87117813.3
(22) Date of filing: 02.12.1987
(51) Int. Cl.: A61B 5/00

(54) **Transcutaneous blood gas sensor**
Transkutaner Blutgasmessfühler
Dispositif de mesure transcutanée de gaz du sang

(30) Priority: 05.12.1986 JP 291358/86
(43) Date of publication of application: 06.07.1988
(73) Proprietor: SUMITOMO ELECTRIC INDUSTRIES, LTD., Osaka-shi, Osaka 541 (JP)
(72) Inventor: Kogo, Katsuyuki c/o Osaka Works, Konohana-ku Osaka (JP)
(74) Representative: Patentanwälte Kirschner & Grosse

(56) References cited:
- EP-A- 0 077 054
- DE-B- 2 919 118
- DE-C- 3 507 183
- US-A- 4 197 853

## Description

### BACKGROUND OF THE INVENTION

### Field of the Invention

The present invention relates to a transcutaneous blood gas sensor, which can continuously measure gas concentration in the blood of the human body by attaching a sensor onto the skin.

### Description of the Prior Art

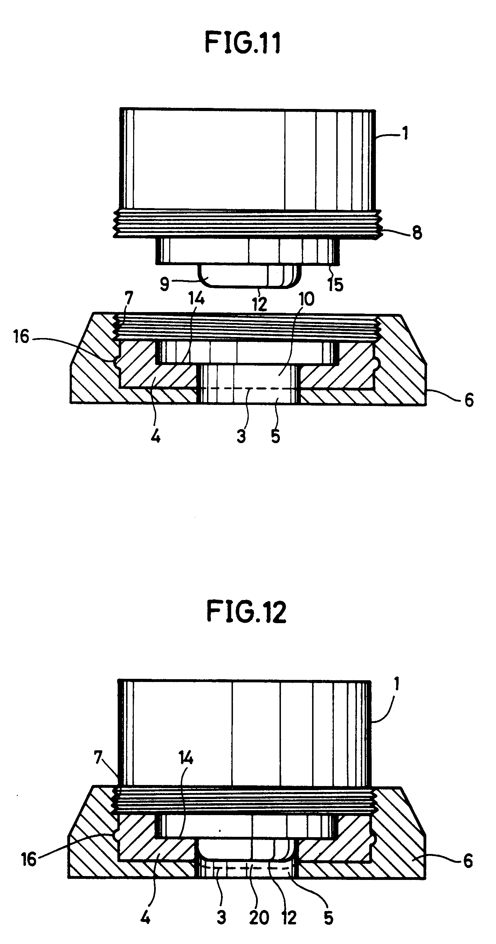

A well-known conventional transcutaneous blood gas sensor is disclosed in Japanese Utility Model Publication Gazette No. 31220/1984, for example. Fig. 11 is a sectional view showing an example of such a conventional transcutaneous blood gas sensor. The conventional gas sensor as shown in Fig. 11 is formed by a sensor body 1, a membrane holder 4 and a ring-shaped collar 6. The membrane holder 4 is provided between the sensor body 1 and the ring-shaped collar 6, which is threadedly connected to the sensor body 1 to mount the membrane holder 4 on the sensor body 1. The sensor body 1 is provided in its periphery with a thread part 8 and the ring-shaped collar 6 is provided in its inner periphery with a thread part 7 for such threaded connection.

The sensor body 1 is further provided in its central portion with a central projection 9, which has a flat end surface 12. The membrane holder 4 is provided in its central portion with a membrane stretcher hole 10 which is sized to fittingly receive the central projection 9 of the sensor body 1, and a membrane 3 is held between the periphery of the membrane stretcher hole 10 and that of a ring part opening 5 of the ring-shaped collar 6. The membrane holder 4 and the ring-shaped collar 6 are fastened to each other at a circumferential irregular part 16.

In application, the sensor body 1 is threadedly connected with the ring-shaped collar 6 so as to retain an electrolytic solution 20 between the membrane 3 and the end surface 12 of the central projection 9. Fig. 12 shows this state. As obvious from Fig. 12, the electrolytic solution 20 is retained between the membrane 3 and the end surface 12 of the central projection 9. In the conventional gas sensor of the aforementioned structure, however, the electrolytic solution is gradually collected to be excessively retained. The electrolytic solution thus excessively retained is moved when the membrane touches the skin in attachment/detachment of the sensor to/from the human body, whereby the measured value of the gas sensor is extremely fluctuated not to return to the original value in most cases, and hence calibration must be again performed.

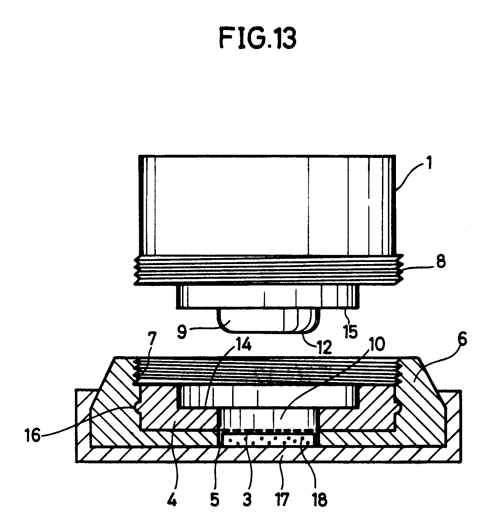

Fig. 13 shows a jig 17 for threaded connection, which jig is well known as means for solving such a problem. The jig 17 is engageable/disengageable with/from the ring-shaped collar 6, and has an elastic member 18 in a part located in the ring part opening 5 of the ring-shaped collar 6. The elastic member 18, being prepared by rubber or sponge, is adapted to press the membrane 3 when the sensor body 1 is threadedly connected with the ring-shaped collar 6. The membrane 3 is so pressed in threaded connection that no excessive electrolytic solution is retained between the membrane 3 and the end surface 12 of the central projection 9. In such a method, however, the jig for threaded connection must be engaged/disengaged with/from the sensor for replacement of the membrane, whereby operation for replacing the membrane is complicated.

From the US-A 4 197 853 a transcutaneous blood gas sensor for a continuous measurement of gas concentration in the blood is known . This conventional sensor comprise a sensor casing with a central projection having a spherical end surface without a given diameter. This spherical end surface is in contact with an electrolytic solution which is retained by a membrane in a chamber between the surface, the membrane and a membrane holder, in which the electrolyte solution is gradually collected to be excessively retained.

The object of the present invention is to provide a transcutaneous blood gas sensor in which the volume of an electrolytic solution retained between a sensor body and a membrane can suitably be adjusted while uniformalizing thickness of the layer of the electrolytic solution without employing a specific jig for threadedly connecting the membrane holder to the sensor body whereby the measured value can be stabilized in a short time after replacement of the membrane.

In the transcutaneous blood gas sensor according to the present invention, a central projection of the sensor body has a spherical end surface, whose radius is at least twice and not more than six times the inner diameter of a membrane stretcher hole.

According to the present invention, the electrolytic solution can be retained in suitable volume between the sensor body and the membrane and the layer of the electrolytic solution can be uniformalized in thickness without employing a jig for threaded connection such as that in the prior art. Thus, no extreme fluctuation of the measured value is caused even if the membrane is touched by the skin or the like during measurement, while the measured value can be stabilized in a short time after replacement of the membrane.

These and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a sectional view showing an embodiment of the present invention in a state previous to threaded connection;
Fig. 2 is a sectional view showing the embodiment of Fig. 1 in an intermediate state of threaded connection;
Fig. 3 is a sectional view showing the embodiment of Fig. 1 in a state after threaded connection;
Fig. 4 is a sectional view schematically showing another embodiment of the present invention;
Fig. 5 illustrates fluctuation of the measured value of reference example, in which a spherical end surface of a central projection of a sensor body has a radius of 1.5 times the inner diameter of a membrane stretcher hole;
Fig. 6 illustrates fluctuation of the measured value of Example of the present invention, in which a spherical end surface of a central projection of a sensor body has a radius of 4 times the inner diameter of a membrane stretcher hole;
Fig. 7 illustrates fluctuation of the measured value of reference example, in which a spherical end surface of a central projection of a sensor body has a radius of 7 times the inner diameter of a membrane stretcher hole;
Fig. 8 illustrates fluctuation of the measured value of reference example, in which a central projection of a sensor body has a flat end surface;
Fig. 9 illustrates fluctuation of the measured value of the reference example as shown in Fig. 5 after replacement of the membrane;
Fig. 10 illustrates fluctuation in the measured value of the Example as shown in Fig. 6 after replacement of the membrane;
Fig. 11 is a sectional view showing a conventional gas sensor in a state previous to threaded connection;
Fig. 12 is a sectional view showing the conventional gas sensor in a state after threaded connection; and
Fig. 13 is a sectional view for illustrating a jig generally employed for threaded connection.

### DESCRIPTION OF THE PREFERRED EMBODIMENTS

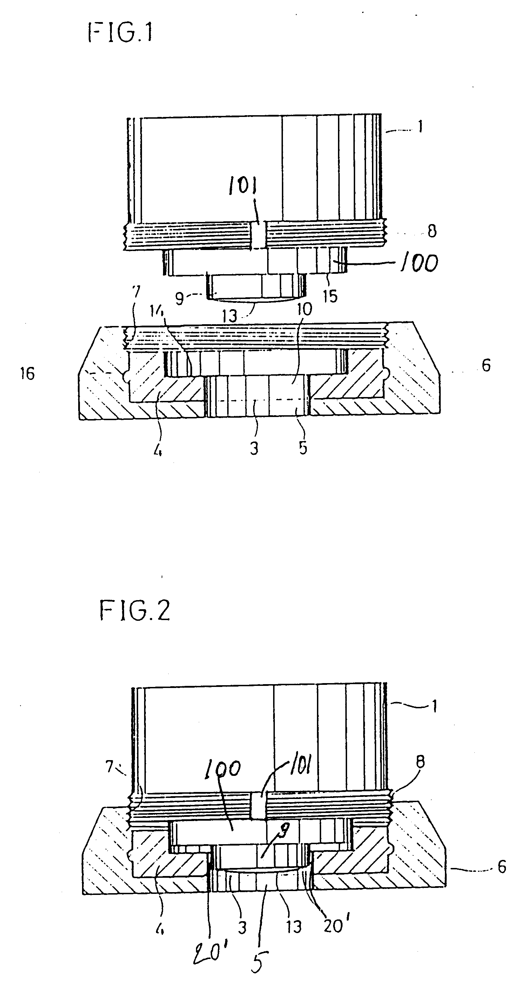

An embodiment of the present invention is now described with reference to Figs. 1 to 3. A membrane holder 4 is provided between a sensor body 1 and a ring-shaped collar 6, which is threadedly connected to the sensor body 1 to mount the membrane holder 4 on the sensor body 1. The sensor body 1 is provided in its periphery with a thread part 8 and the ring-shaped collar 6 is provided in its inner peripheral portion with a thread part 7, for such threaded connection.

The sensor body 1 is further provided in its central portion with a central projection 9, which has a spherical end surface 13. The radius of the spherical end surface 13 is at least twice and not more than six times the inner diameter of a membrane stretcher hole 10. The membrane holder 4 is provided in its central portion with the membrane stretcher hole 10, which is sized to fittingly receive the central projection 9 of the sensor body 1. A membrane 3 is held between the periphery of the membrane stretcher hole 10 and the periphery of a ring part opening 5 of the ring-shaped collar 6. The membrane holder 4 and the ring-shaped collar 6 are fastened to each other at a circumferential irregular part 16.

An abutting surface 15 is formed around the central projection 9 of the sensor body 1, while an abutting surface 14 is formed around the membrane stretcher hole 10 of the membrane holder 4. These abutting surfaces 15 and 14 are oppositely brought into contact with each other when the ring-shaped collar 6 and the membrane holder 4 are mounted on the sensor body 1, thereby to prevent leakage of an electrolytic solution retained between the end surface 13 of the central projection 9 and the membrane 3.

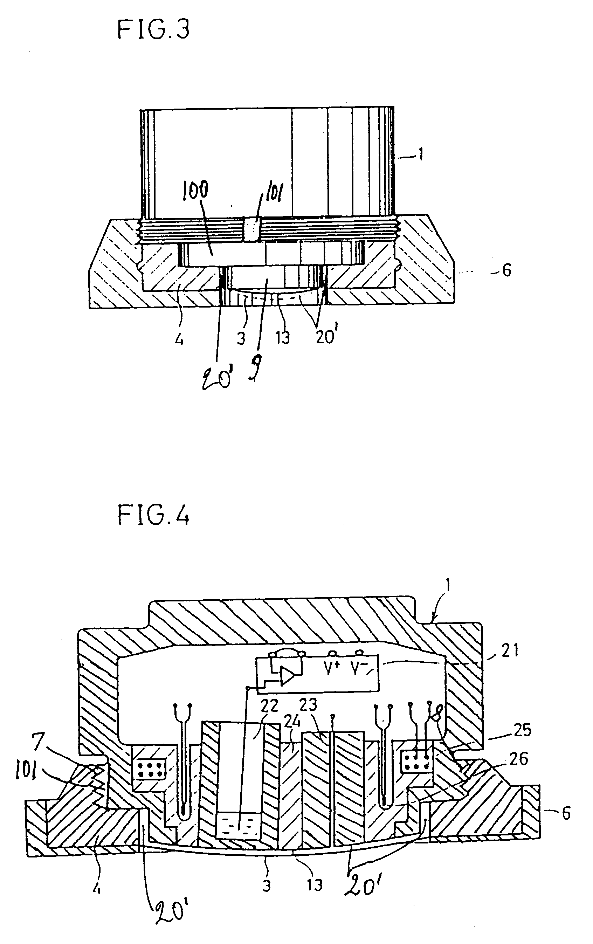

Fig. 2 is a sectional view showing a state in which the ring-shaped collar 6 is being threadedly connected to the sensor body 1, and Fig. 3 is a sectional view showing a state after threaded connection. In operation for such threaded connection, the spherical end surface 13 is first brought into contact with the membrane 3 as shown in Fig. 2, so that the contact portion between the end surface 13 and the membrane 3 is gradually enlarged as the end surface 13 is downwardly moved. Thus, the electrolytic solution is gradually circumferentially spread to flow out through the thread parts 7 and 8. In order to smooth such outflow of the electrolytic solution, the thread part 8 of the sensor body 1 may be provided with grooves which are perpendicular to the thread ridges.

Thus, the electrolytic solution is discharged upon threaded connection, whereby no excessive electrolytic solution is retained between the membrane 3 and the end surface 13, as shown in Fig. 3.

According to the present invention, the spherical end surface of the central projection of the sensor body has the radius of at least twice and not more than six times the inner diameter of the membrane stretcher hole since the effect of circumferentially spreading the electrolytic solution cannot be sufficiently attained if the radius of the spherical end surface is greater than six times the inner diameter of the membrane stretcher hole. On the other hand, the membrane is pressed and expanded by the end surface of the central projection if the said radius is less than twice the said inner diameter, whereby gas permeability thereof is gradually changed to require a considerable time for returning to a steady state. The measured value of the sensor is stabilized within 30 minutes after threaded connection in the scope of the present invention, in which the radius of the spherical end surface is at least twice the inner diameter of the membrane stretcher hole. However, several hours are required for such stabilization if the said radius is smaller than twice the said inner diameter.

Fig. 4 is a sectional view schematically showing another embodiment (Example A) of the present invention. Referring to Fig. 4, a gas sensor of Example A was formed by a preamplifier 21, a pH electrode 22 for measuring carbon dioxide gas, a cathode 23 for measuring oxygen, a silver electrode 24 (hatched in Fig. 4), a heater 25 and a thermistor 26. Other structure was similar to that of Fig. 1, and hence redundant description is omitted. The silver electrode 24 was adapted to serve as a skin heater, an anode for measuring oxygen and a reference electrode for measuring carbon dioxide gas. In the gas sensor as shown in Fig. 4, a membrane stretcher hole of a membrane holder was 10 mm in inner diameter. A spherical end surface of a central projection of a sensor body was 40 mm in radius, within the scope of the present invention. For the purpose of comparison, gas sensors having spherical end surfaces of 15 mm and 70 mm in inner diameter, being out of the scope of the present invention, and a gas sensor including a central projection having a flat end surface were prepared as reference examples B, C and D respectively.

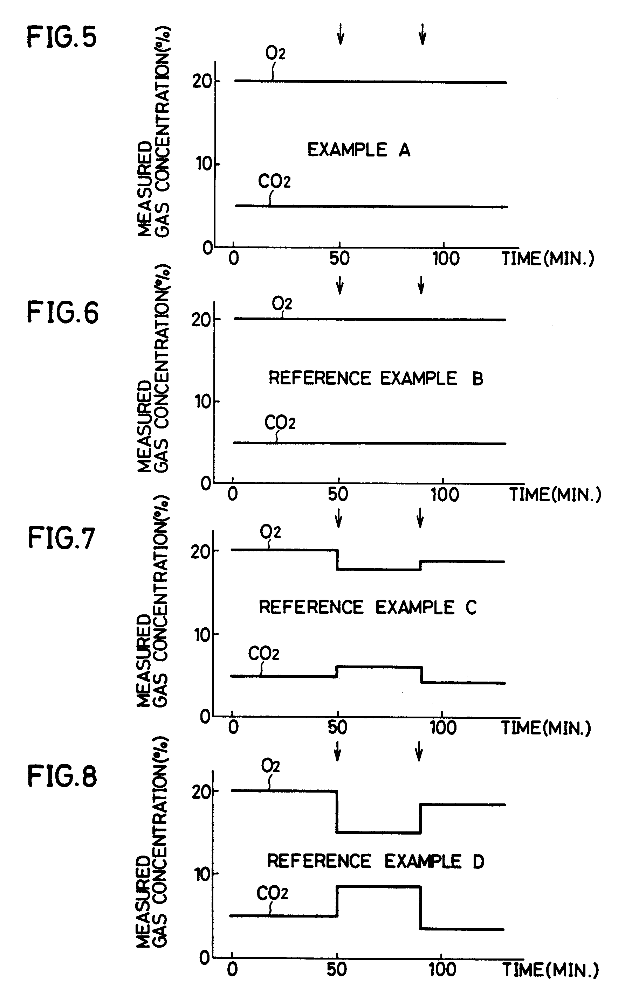

These four types of gas sensors were left in a gas atmosphere, being composed of 5 percent of CO₂, 20 percent of O₂ and 75 percent of N₂, to measure CO₂ concentration and O₂ concentration. CO₂ concentration of 5 percent and O₂ concentration of 20 percent were set previous to starting of data recording, and the membranes of the gas sensors were touched by a finger upon lapses of 50 minutes and 90 minutes from starting of data recording. Figs. 5 to 8 illustrates the states of fluctuation in measured values (downward arrows indicate finger touches). In reference example B as shown in Fig. 5 and Example A as shown in Fig. 6, substantially no fluctuation of the measured values was recognized in finger touches to the membranes. On the other hand, the measured values were extremely fluctuated when the membranes were touched in reference examples C and D as shown in Figs. 7 and 8.

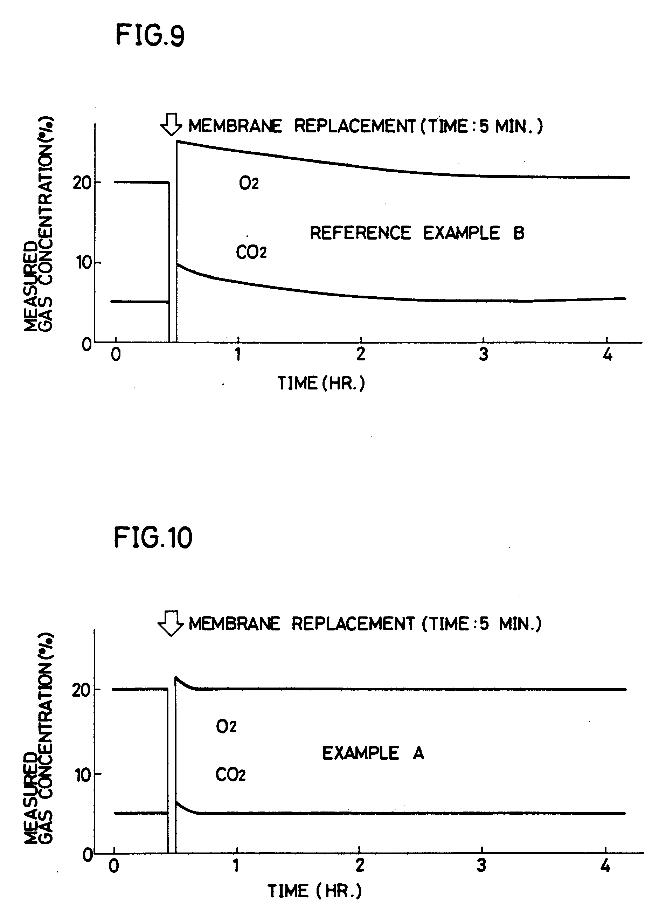

Fluctuation of the measured values in replacement of the membranes were checked with respect to Example A and reference example B, whose measured values were not fluctuated by finger touches to the membranes, i.e., whose measured values were stable. Figs. 9 and 10 show the results of measurement of reference example B and Example A respectively. As obvious from Figs. 9 and 10, several hours were required for stabilization of the measured value after replacement of the membrane in reference example B, whose central projection had a spherical end surface of a radius out of the scope of the present invention, while the measured value was stabilized within about 10 minutes after replacement of the membrane in Example A, whose central projection had a spherical end surface of a radius within the scope of the present invention. Thus, it is obvious that the time required for stabilization of the measured value is reduced as the radius of the spherical end surface of the central projection is increased. The measured value is preferably stabilized within about 30 minutes in practice, and hence the spherical end surface of the central projection must have a radius of greater than twice the inner diameter of the membrane stretcher hole.

As hereinabove described, the spherical end surface of the central projection has a radius of at least twice and not more than six times the inner diameter of the membrane stretcher hole, so that the measured value is not fluctuated even if the membrane is touched by a finger and the measured value can be stabilized in a short time after replacement of the membrane. The fluctuation in the measured value caused by a touch to the membrane comes into question particularly when the inner diameter of the membrane stretcher hole is in excess of 4 mm, since the membrane is frequently touched by the skin or the like in this case.

The excessive electrolytic solution must be discharged in threaded connection of the sensor body and the ring-shaped collar. In order to ensure such outflow of the electrolytic solution, the thread part of the sensor body is provided with grooves which are perpendicular to the thread ridges, as hereinabove described. Although the membrane holder and the ring-shaped collar are threadedly mounted on the sensor body in the aforementioned embodiment, the method of such mounting is not restricted to threaded connection according to the present invention, but another mounting method such as fastening can be employed.

While the membrane holder is previously assembled into the ring-shaped collar in the aforementioned embodiment, the present invention is not restricted to such process. Further, the abutting surfaces are provided in the peripheries of the central projection of the sensor body.

The gas measuring part of the sensor body may be adapted to measure either oxygen gas, carbon dioxide or other gas.

## Claims

1. A transcutaneous blood gas sensor comprising a membrane (3), sensor means for sensing gas, comprising a sensor housing (1) with a central projection (9) having a portion forming a spherical segment for contacting said membrane, said spherical segment having a spherical end surface (13), a ring-shaped collar (6) to be attached to a patient's skin, a membrane holder (4) having a membrane stretcher hole (10) for receiving said central projection (9) of said sensor housing (1), said membrane holder (4) cooperating with said ring-shaped collar (6) for retaining said membrane (3) across said membrane stretcher hole (10) between said membrane holder (4) and said ring-shaped collar (6), said spherical end surface (13), said membrane (3), a peripheral surface of said central projection (9) and an inner surface of said membrane stretcher hole (10) forming a chamber (20') for holding an electrolytic solution, said chamber (20') having a chamber bottom portion between said membrane (3) and said spherical end surface (13) and a chamber ring portion between said membrane holder (4) and said central projection (9) so that said central projection is surrounded by a sufficient volume of electrolytic solution, means (7,8) for operatively holding said membrane holder (4) in place, **characterized in that**
said stretcher hole (10) having a given hole diameter, said spherical end surface (13) having a radius of spherical curvature corresponding in length to a multiple of said given hole diameter of said stretcher hole (5), said multiple being within the range of at least two to not more than six, groove means (101) being formed in a threaded part of the sensor body perpendicular to threaded ridges for providing a release path for excessive electrolytic solution out of said chamber (20') when said sensor housing (1), said membrane holder (4) and said ring-shaped collar (6) are being brought into an assembled position relative to one another, and abutting surfaces (14, 15) for preventing leakage along said release path when said assembled position housing has been established.

2. Sensor according to claim 1, characterized in that said given hole diameter of said membrane stretcher hole (5) is at least 4mm.

3. Sensor according to claims 1 or 2, characterized in that said means for closing said release path comprises a stepped connecting section (100) in said sensor housing (1), said stepped connecting section (100) including a first abutting ring surface (15) around said central projection (9), said membrane holder (4) having a second abutting ring surface (14) surrounding said stretcher hole (5), said first and second abutting surfaces (15, 14) contacting each other to close said release path when said assembled position has been established.

4. Sensor according to one of claims 1 to 3, characterized in that said membrane (3) has a central portion stretched across said stretcher hole (5) and a ring portion surrounding said central membrane portion, said membrane ring portion extending radially and flat away from said central membrane portion, said membrane ring portion being clamped between said ring-shaped collar (6) and said membrane holder (4).

5. Sensor according to one of claims 1 to 4, characterized in that said means for operatively holding said membrane holder (4) in place comprise threading means (7, 8) provided on said sensor housing (1) and on said ring-shaped collar (6) whereby said membrane holder (4) is held in place between said sensor housing (1) and said ring-shaped collar (6).

6. Sensor according to one of claims 1 to 5, characterized in that said means for operatively holding said membrane holder in place comprise threading means (7, 8) provided on said sensor housing (1) and on said membrane holder (4), whereby said membrane holder (4) is threaded to said sensor housing (1), and wherein said ring-shaped collar (6) is secured to said membrane holder (4).

7. Sensor according to one of claims 1 to 6, characterized in that a gas measuring part of said sensor body is adapted to measure oxygen gas.

8. Sensor according to one of claims 1 to 6, characterized in that a gas measuring part of said sensor body is adapted to measure carbon dioxide gas.

9. Sensor according to one of claims 1 to 6, characterized in that a gas measuring part of said sensor body is adapted to measure oxygen gas and carbon dioxide gas.

## Patentansprüche

1. Tanskutaner Blutgasmeßfühler umfassend eine Membran (3), einen Fühler zum Erfassen von Gas, der ein Fühlergehäuse (1) mit einem mittigen Vorsprung (9) mit einem Abschnitt umfassen, der ein sphärisches Segment zur Kontaktierung der Membran bildet, wobei das sphärische Segment eine sphärische Endoberfläche (13) hat, eine ringförmige Manschette (6), die auf der Haut des Patienten angebracht wird, einen Membranhalter (4) mit einem Membranspannloch (10) zur Aufnahme des mittigen Vorsprungs (9) des Fühlergehäuses (1), wobei der Membranhalter (4) mit der ringförmigen Manschette (6) zum Festhalten der Membran (3) über dem Membranspannloch (10) zwischen dem Membranhalter (4) und der ringförmigen Manschette (6) zusammenwirkt, wobei die sphärische Endoberfläche (13), die Membran (3), eine am Rand befindliche Oberfläche des mittigen Vorsprungs (9) und eine innere Oberfläche des Membranspannlochs (10) eine Kammer (20') zum Halten einer elektrolytischen Lösung bilden, wobei die Kammer (20') einen Kammerbodenabschnitt zwischen der Membran (3) und der sphärischen Endoberfläche (13) und ein Kammerringteil zwischen dem Membranhalter (4) und dem mittigen Vorsprung (9) hat, sodaß der mittige Vorsprung von einem ausreichenden Volumen elektrolytischer Lösung umgeben ist, und Mittel (7, 8), um den Membranhalter (4) wirksam an der Stelle zu halten, dadurch gekennzeichnet, daß das Spannloch (10) einen vorgegebenen Lochdurchmesser hat, daß die sphärische Endoberfläche (13) einen sphärischen Krümmungsradius hat, dessen Betrag einem Vielfachen des vorgegebenen Lochdurchmessers des Spannlochs (10) entspricht, daß das genannte Vielfache innerhalb des Bereichs von mindestens zwei und nicht mehr als sechs liegt, wobei Nuten (101) in einem Gewindeteil des Fühlerkörpers senkrecht zu den Gewindegraten gebildet sind, um einen Auslaßweg für überschüssige, elektrolytische Lösung aus der Kammer (20') zu bilden, wenn das Fühlergehäuse (1), der Membranhalter (4) und die ringförmige Manschette (6) relativ zueinander in eine zusammengesetzte Stellung gebracht wurden, und wobei Anschlag-Oberflächen (14, 15) zur Vermeidung von Lecks längs dem Auslaßweg gebildet sind, wenn das Gehäuse in der zusammengesetzten Stellung ist.

2. Blutgasmeßfühler nach Anspruch 1, dadurch gekennzeichnet, daß der vorgegebene Lochdurchmesser des Membranspannlochs (5) mindestens 4 mm ist.

3. Blutgasmeßfühler nach Anspruch 1 und 2, dadurch gekennzeichnet, daß die Mittel zum Schließen des Auslaßwegs einen gestuften Verbindungsabschnitt (100) in dem Fühlergehäuse (1) umfassen, wobei der gestufte Verbindungsabschnitt (100) eine erste Anschlag-Ringoberfläche (15) um den mittigen Vorsprung (9) umfaßt, wobei der Membranhalter (4) eine zweite Anschlag-Ringoberfläche (14) um das Spannloch (5) hat, wobei die erste und die zweite Anschlag-Oberfläche (15, 14) miteinander in Kontakt gebracht werden, um den Auslaßweg zu schließen, wenn die zusammengesetzte Stellung verwirklicht ist.

4. Blutgasmeßfühler nach einem der Ansprüche 1 bis 3, dadurch gekennzeichnet, daß die Membran (3) ein mittiges über das Spannloch (5) gespanntes Stück und ein das mittiges Membranstück umgebendes Ringstück hat, wobei sich das Membranringstück radial und flach vom dem mittigen Membranstück fortsetzt, wobei das Membranringstück mit Klammern zwischen der ringförmiger Manschette (6) und dem Membranhalter (4) befestigt ist.

5. Blutgasmeßfühler nach einem der Ansprüche 1 bis 4, dadurch gekennzeichnet, daß die Mittel, um den Membranhalter (4) wirksam in seiner Lage zu halten, Gewindeteile (7, 8) umfassen, die auf dem Fühlergehäuse (1) und auf der, ringförmigen Manschette (6) vorgesehen sind, wobei der Membranhalter (4) in Stellung zwischen dem Fühlergehäuse (1) und der, ringförmige Manschette (6) gehalten wird.

6. Blutgasmeßfühler nach einem der Ansprüche 1 bis 5, dadurch gekennzeichnet, daß die Mittel, den Membranhalter (4) wirksam in seiner Lage zu halten, Gewindeteile (7, 8) umfassen, die auf dem Fühlergehäuse (1) und auf dem Membranhalter (4) vorgesehen sind, wobei der Membranhalter (4) mit dem Fühlergehäuse (1) verschraubt ist, und daß die ringförmige Manschette (6) an dem Membranhalter (4) befestigt ist.

7. Blutgasmeßfühler nach einem der Ansprüche 1 bis 6, dadurch gekennzeichnet, daß ein Gas-Meßteil des Fühlerkörpers zur Messung von Sauerstoffgas angepaßt ist.

8. Blutgasmeßfühler nach einem der Ansprüche 1 bis 6, dadurch gekennzeichnet, daß ein Gas-Meßteil des Fühlerkörpers zur Messung von Kohlenstoffdioxidgas angepaßt ist.

9. Blutgasmeßfühler nach einem der Ansprüche 1 bis 6, dadurch gekennzeichnet, daß ein Gas-Meßteil des Fühlerkörpers zur Messung von Sauerstoffgas und Kohlenstoffdioxidgas angepaßt ist.

## Revendications

1. Détecteur transcutané de gaz dans le sang, comprenant une membrane (3), un moyen détecteur pour détecter le gaz, comprenant un boîtier de détecteur (1) avec une saillie centrale (9) ayant une partie formant un segment sphérique pour venir en contact avec ladite membrane, ledit segment sphérique ayant une surface d'extrémité sphérique (13), une embase annulaire (6) à fixer sur la peau d'un patient, un porte-membrane (4) ayant un trou tendeur de membrane (10) pour recevoir ladite saillie centrale (9) dudit boîtier de détecteur (1), ledit porte-membrane (4) coopérant avec ladite embase annulaire (6) pour maintenir ladite membrane (3) en travers dudit trou tendeur de membrane (10) entre ledit porte-membrane (4) et ladite embase annulaire (6), ladite surface d'extrémité sphérique (13), ladite membrane (3), une surface périphérique de ladite saillie centrale (9) et une surface interne dudit trou tendeur de membrane (10) formant une chambre (20') pour contenir une solution électrolytique, ladite chambre (20') ayant une partie de chambre inférieure entre ladite membrane (3) et ladite surface d'extrémité sphérique (13) et une partie de chambre annulaire entre ledit porte-membrane (4) et ladite saillie centrale (9) de telle manière que ladite saillie centrale est entourée par un volume suffisant de solution électrolytique, et des moyens (7, 8) pour maintenir en place ledit porte-membrane (4) en fonctionnement, caractérisé en ce que ledit trou tendeur de membrane (10) a un diamètre de trou donné, ladite surface d'extrémité sphérique (13) a un rayon de courbure sphérique correspondant en longueur à un multiple dudit diamètre de trou donné dudit trou tendeur (5), ledit multiple étant situé dans une plage d'au moins deux jusqu'à six maximum, en ce qu'un moyen de rainure (101) est formé dans une partie filetée du corps de détecteur perpendiculairement aux sillons du filetage, pour constituer une voie d'évacuation pour le surplus de solution électrolytique hors de ladite chambre (20') lorsque ledit boîtier de détecteur (1), ledit porte-membrane (4) et ladite embase annulaire (6) sont amenés en position assemblée les uns par rapport aux autres, et que des surfaces (14, 15) sont en contact pour prévenir une fuite le long de ladite voie d'évacuation lorsque ladite position assemblée du boîtier a été établie.

2. Détecteur selon la revendication 1, caractérisé en ce que ledit diamètre de trou donné dudit trou tendeur de membrane (5) est d'au moins 4 mm.

3. Détecteur selon les revendications 1 ou 2, caractérisé en ce que ledit moyen pour fermer ladite voie d'évacuation comprend une partie intermédiaire en gradin (100) dans ledit boîtier de détecteur (1), ladite partie intermédiaire en gradin (100) comprenant une première surface annulaire de contact (15) autour de ladite saillie centrale (9), ledit porte-membrane (4) ayant une seconde surface annulaire de contact (14) entourant ledit trou tendeur (5), lesdites première et seconde surfaces de contact (15, 14) venant en contact entre elles pour fermer ladite voie d'évacuation lorsque ladite position assemblée a été établie.

4. Détecteur selon l'une quelconque des revendications 1 à 3, caractérisé en ce que ladite membrane (3) a une partie centrale tendue en travers dudit trou tendeur (5) et une partie annulaire entourant ladite partie centrale de membrane, ladite partie annulaire de membrane s'étendant radialement et à plat en s'éloignant de ladite partie centrale de membrane, ladite partie annulaire de membrane étant serrée entre ladite embase annulaire (6) et ledit porte-membrane (4).

5. Détecteur selon l'une quelconque des revendications 1 à 4, caractérisé en ce que lesdits moyens pour maintenir en place ledit porte-membrane (4) en fonctionnement comprennent des moyens de filetage (7, 8) prévus sur ledit boîtier de détecteur (1) et sur ladite embase annulaire (6), permettant de maintenir en place ledit porte-membrane (4) entre ledit boîtier de détecteur (1) et ladite embase annulaire (6).

6. Détecteur selon l'une quelconque des revendications 1 à 5, caractérisé en ce que lesdits moyens pour maintenir en place ledit porte-membrane (4) en fonctionnement comprennent des moyens de filetage (7, 8) prévus sur ledit boîtier de détecteur (1) et sur ledit porte-membrane (4), ledit porte-membrane (4) étant ainsi vissé sur ledit boîtier de détecteur (1), et ladite embase annulaire (6) étant fixée sur ledit porte-membrane (4).

7. Détecteur selon l'une quelconque des revendications 1 à 6, caractérisé en ce qu'un élément de mesure de gaz dudit corps de détecteur est propre à mesurer l'oxygène.

8. Détecteur selon l'une quelconque des revendications 1 à 6, caractérisé en ce qu'un élément de mesure de gaz dudit corps de détecteur est propre à mesurer l'anhydride carbonique.

9. Détecteur selon l'une quelconque des revendications 1 à 6, caractérisé en ce qu'un élément de mesure de gaz dudit corps de détecteur est propre à mesurer l'oxygène et l'anhydride carbonique.
